# EUROPÄISCHE PATENTSCHRIFT

(11) **EP 1 406 615 B1**
(45) Veröffentlichungstag und Bekanntmachung des Hinweises auf die Patenterteilung: **03.11.2010**
(21) Anmeldenummer: 02762351.1
(22) Anmeldetag: 12.07.2002
(51) Int. Cl.: A61K 31/404, C07D 209/34, A61P 35/00

(54) **KINASE INHIBITOREN UND DEREN VERWENDUNG**
KINASE INHIBITORS AND THE USE THEREOF
INHIBITEURS DE KINASE ET LEUR UTILISATION

(30) Priorität: 13.07.2001 DE 10134196
(43) Veröffentlichungstag der Anmeldung: 14.04.2004
(73) Patentinhaber: Karlsruher Institut für Technologie, 76131 Karlsruhe (DE)
(72) Erfinder: CHIRCHIN, Vladimir, 60596 Frankfurt/Main (DE); ATHANASSIOS, Giannis, 04316 Leipzig (DE); MAZITSCHEK, Ralph, 76137 Karlsruhe (DE); SLEEMAN, Jonathan, 76646 Bruchsal (DE)
(74) Vertreter: Fitzner, Uwe
(86) Internationale Anmeldenummer: PCT/EP2002/007778
(87) Internationale Veröffentlichungsnummer: WO 2003/007943

(56) Entgegenhaltungen:
- EP-A- 0 632 102
- WO-A-96/40116
- WO-A-98/07695
- WO-A-99/10325
- BLUM ET AL.: "Substrate competitive inhibitors of IGF-1 receptor kinase" BIOCHEMISTRY, Bd. 39, Nr. 51, 2000, Seiten 15705-12, XP002215776
- DATABASE CROSSFIRE BEILSTEIN [Online] Beilstein Institut zur Förderung der Chemischen Wissenschaften, Frankfurt am Main, DE; retrieved from BEILSTEIN, accession no. 231732 XP002215778 & WAHL; BAGARD: BULL. SOC. CHIM. FR. , Bd. 4, Nr. 5, 1909, Seite 1038
- HAMADA K ET AL: "VEGF-C SIGNALING PATHWAYS THROUGH VEGFR-2 AND VEGFR-3 IN VASCULOANGIOGENESIS AND HEMATOPOIESIS" BLOOD, W.B.SAUNDERS COMPAGNY, ORLANDO, FL, US, Bd. 12, Nr. 96, 2000, Seiten 3793-3800, XP002952145 ISSN: 0006-4971
- V. KIRKIN ET AL.: "Characterization of indolinones which preferentially inhibit VEGF-C and VEGF-D-induced activation of VEGFR-3 rather than VEGFR-2" EUR. J. BIOCHEM., Bd. 268, November 2001 (2001-11), Seiten 5530-40, XP002215777

## Beschreibung

Die vorliegende Erfindung betrifft Mittel zur Blockierung der Aktivität von Rezeptor-Tyrisin-Kinasen aus der Gruppe VEGFR-3 (Vascular Endothelial Cell Growth Factor Receptor) und/oder VEGFR-2 und deren Verwendung zur Behandlung von Krankheiten, die durch pathologische Signaltransduktionskaskaden ausgelöst wurden.

Proteinkinasen sind zu den Transferasen gehörende Enzyme, welche die Übertragung von Phosphatresten des Adenosin-5'-Triphosphats (ATP) oder Guanosin-5'-Triphosphats (GTP) auf Proteine katalysieren. Nach den Aminosäure-Resten, auf die die Phosphatgruppe übertragen wird, unterscheidet man z. B. Protein-Serin/Threonin-Kinasen, Protein-Hisitidin-Kinasen, Protein-Aspartat-Kinasen oder Protein-Tyrosin-Kinasen.

Proteinkinasen spielen eine entscheidende Rolle in der Regulierung der Aktivität der Akzeptorproteine (Signaltransduktionskaskade). Signale von außerhalb der Zelle werden durch Zelloberflächen-Rezeptoren, wie z. B. Rezeptor-Tyrosin-Kinasen (RTKs) aufgenommen (Ullrich et al. 1990, Cell, 61: 203-212; Fantl et al., 1993, Annu. Rev. Biochem., 62: 453-481). Durch die Bindung von "signalgebenden" Molekülen oder sogenannten Effektoren oder Liganden, werden die RTKs autophosphoryliert (Weiss et al., 1997, Curr. Opin. Genet. Div., 7:80-86). Diese Autophosphorylierung erlaubt es den RTKs mit anderen Proteinen, u.a. sogenannten Adapter-Proteinen eine Wechselwirkung einzugehen (Robertson et al., 2000, Trends Genet., 16: 268-271). Diese Proteinkomplexe sind ihrerseits in der Lage, andere intrazelluläre Proteine zu aktivieren, was zu einer ganzen Kette von Proteininteraktionen führt, wodurch das ursprünglich extrazelluläre Signal von der Zelloberfläche bis in den Zellkern übertragen wird (Treisman et al., 1996, Curr. Opin. Cell. Biol., 8:205-215; Tan et al., 1999, Trends Genet., 15:1456-149). Das übertragene Signal ist somit in der Lage, die Genexpression, den Zellzyklus oder andere wichtige Zellfunktionen zu beeinflussen.

Zu den Effektoren der Rezeptor-Tyrosin-Kinasen gehören beispielsweise Insulin und viele Wachstumsfaktoren, wie z. B. die Wachstumsfaktoren der Blutplättchen (PDGF) oder epidermale Wachstumsfaktoren (EGF). Rezeptor-Tyrosin-Kinasen spielen u.a. eine wichtige Rolle bei der Regulierung der Bildung neuer Blutgefäße (Angiogenese) oder neuer lymphatischer Gefäße (Lymphangiogenese). Hierbei werden Endothelzellen von bereits existierenden Gefäßen angeregt, zu wachsen, zu sprossen und sich auszudehnen, um neue Kapillarfgefäße zu bilden. In diesem Zusammenhang sind insbesondere die Zelloberflächen-Rezeptoren VEGFR (vascular endothelial growth factor receptor) und FGFR (fibroblast growth factor receptor) sowie als Effektoren entsprechende Wachstumsfaktoren der VEGF-Familie oder FGF-Familie zu nennen (Korpelainen et al., 1998, Curr. Opin. Cell. Biol., 10: 159-164; Malonne et al., 1999, Clin. Exp. Metastasis., 17:1-14). Weitere bekannte Beispiele für natürliche angiogene Effektoren (Liganden) sind u.a. der Tumor-Necrosis-Faktor (TNF-α), Interleukin 8 oder der sogenannte Tie₂-Ligand (Malonne et al., 1999, Clin. Exp. Metastasis., 17:1-14).

Die unkontrollierte Stimulierung von Proteinkinasen kann zu pathologischen Prozessen, wie z. B. Krebs führen (Porter et al., 1998, Oncogene, 17: 1343-1352). Beispielsweise kann ein genetisch veränderter Rezeptor, also eine mutierte Rezeptor-Tyrosin-Kinase, die auch in Abwesenheit eines geeigneten Effektors konstitutiv Signale an andere Proteine weiterleitet, zur Entstehung von Krebs führen. Solche Aktivierungsmutationen von RTKs sind mit einer Vielzahl menschlicher Erkrankungen verknüpft (Robertson et al., 2000, Trends Genet., 16: 268-271). So sind beispielsweise konstitutiv aktive FGF-Rezeptoren für eine Vielzahl von Erbkrankheiten verantwortlich (Tabelle 1). Die inkorrekte Regulation der Angiogenese spielt eine wichtige Rolle im Verlauf einer großen Anzahl von Krankheiten, die in Tabelle 2 aufgeführt sind (Malonne et al., 1999, Clin. Exp. Metastasis., 17:1-14). So haben im Fall von Krebserkrankungen verschiedene Studien ergeben, daß Tumoren in kritischer Weise von einer ausreichenden Blutversorgung abhängig sind. Wenn die Angiogenese gehemmt werden kann, kann auch das Tumorwachstum gestoppt oder sogar revertiert werden (Zetter et al., 1998, Annu. Rev. Med., 49: 407-424). Auch die Induktion der Lymphangiogenese spielt eine wichtige Rolle bei Krebserkrankungen und Filariasis (Skobe et al., 2000, Nature Med., 7: 192-198; Rao et al., 1996, J. Parasitol., 82: 550-556).

Die Regulation der Proteinkinase-Aktivität von Rezeptor-Tyrosin-Kinasen kann im Prinzip auf unterschiedliche Weise erfolgen. So können beispielsweise Antikörper eingesetzt werden, welche die Interaktion der Rezeptor-Kinase/Liganden-Bindung blockieren (Brekken et al., 2000, Cancer Res., 60: 5117-5124; Klement et al., 2000, J. Clinic. Invest., Vol. 105, No. 8: 15-24). Alternativ ist der Einsatz löslicher extrazellulärer Rezeptorabschnitte zur Bindung des entsprechenden Liganden in einen inaktiven Komplex (Sequestrierung) bei Aiello et al. (1995, Proc. Natl. Acad. Sci, Vol. 92: 10457-10461) beschrieben. Sowohl die zuvor genannten Antikörper als auch die löslichen Rezeptoranteile weisen jedoch erhebliche Nachteile auf. Beide werden schnell aus dem Kreislaufsystem entfernt. Ferner handelt es sich in beiden Fällen um sehr große Moleküle, deren Fähigkeit zur Gewebepenetration sehr limitiert ist. Ihre Herstellung, insbesondere die der Antikörper, für die pharmazeutische Anwendung ist sehr aufwendig und teuer. Darüber hinaus repräsentieren sie Verbindungen, die eine Immunantwort auslösen können, so daß ihre biologische Effektivität stark abnimmt bzw. gänzlich außer Kraft gesetzt wird.

Eine weitere Möglichkeit die Aktivität von Protein-Kinasen zu regulieren, ist die Hemmung durch Substrat-ähnliche Verbindungen, die beispielsweise mit dem natürlichen Substrat ATG oder GTP um die Substratbindedomäne konkurrieren. In diesem Zusammenhang sind Indolinone beschrieben, die zur Hemmung von Rezeptor-Tyrosin-Kinasen (RTKs) fähig sind. Kristallographische Studien an der speziellen RTK Fibroblast Growth Factor Receptor (FGFR) haben gezeigt, daß der Oxindol-Anteil der Indolinone mit der gleichen Bindungsstelle interagieren, wie der Adeninring des natürlichen Substrats ATP (Mohammadi, M. et al., Science, 276: 955-960). Allerdings bestimmt sie chemische Struktur des Substituenten am C3-Atom des Oxindols darüber, welche RTK in ihrer Aktivität gehemmt wird. Einige Indolinone blockieren die Aktivität einer einzigen RTK; andere Inolinone hemmen ein breiteres RTK-Spektrum. Indolin-Derivate die ebenfalls als Proteinkinase-Inhibitoren eigesetzt werden, sicd aus der WO 99/10325 A, WO 96/40116 A, WO 98/07695 A sowie von Blum et al. (Biocheistry, Bd. 39, Nr. 51, 2000, Seiten 15705-12) bekannt.

Zur Behandlung von Krankheiten, bei denen die Aktivität von Proteinkinasen krankhaft außer Kontrolle geraten ist, ist es daher von enormer medizinischer Relevanz, Verbindungen zur Verfügung zu stellen, mit denen diese unkontrollierte Aktivität von Oberflächen-Rezeptor-Proteinkinasen, vorteilhafterweise Rezeptor-Tyrosin-Kinasen (RTKs) reguliert, bevorzugt blockiert werden kann, um dadurch den Verlauf der Erkrankungen zu mindern oder sogar zu unterbinden. Krankheiten, bei denen die Aktivität von Proteinkinasen krankhaft außer Kontrolle geraten ist, können z. B. Krebsarten sein, die durch eine unkontrollierte Zellvermehrung und/oder gestörte Apoptose hervorgerufen werden. Ferner kann es sich um Krankheiten wie z. B. Filariasis handeln oder um Krankheiten, die durch gestörte Vorgänge bei der Angiogenese und/oder Lymphangiogenese hervorgerufen werden (siehe dazu auch Tabelle 2). Wünschenswert ist in diesem Zusammenhang auch die Bereitstellung von Verbindungen, die über die natürliche Aktivität oder krankhaft veränderte (konstitutive) Aktivität von Proteinkinasen direkt die Angiogenese und/oder Lymphangiogenese blockieren, so daß einerseits die Blutversorung des Tumors verschlechtert oder sogar unterbunden wird, wodurch es zu einer Eindämmung des Tumorwachstums oder einem Absterben des krankhaften Gewebes kommt bzw. andererseits eine Metastasierung von Tumorzellen verhindert wird.

Diese Aufgabe wird durch die vorliegende Erfindung in vorteilhafter Weise gelöst.

Gegenstand der vorliegenden Erfindung sind Mittel zur Blockierung der Aktivität von Rezeptor-Tyrisin-Kinasen aus der Gruppe VEGFR-3 (Vascular Endothelial Cell Growth Factor Receptor) und/oder VEGFR-2 enthaltend wenigstens eine Verbindungen gemäß der Strukturformel
I) 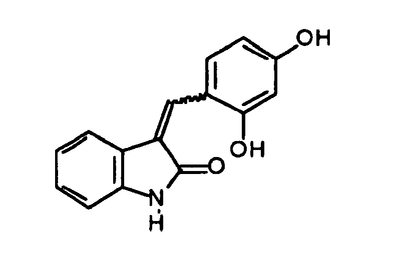 oder
II) 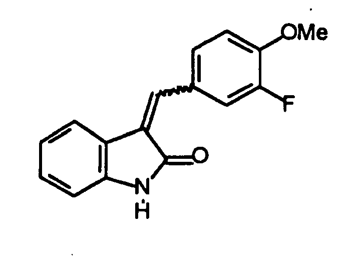 oder
III) 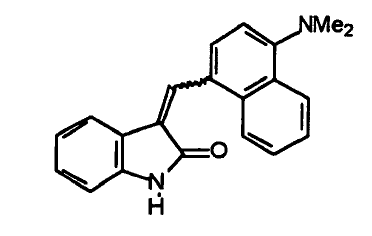
oder Salzen der Verbindungen I) - III).

Diese Verbindungen werden im Laufe der Beschreibung auch mit MAE87 (= Strukturformel I), MAE106 (= Strukturformel II), MAZ51 (= Strukturformel III) bezeichnet. Gegenstand der vorliegenden Erfindung ist ferner eine Verbindung mit der Bezeichnung MAZ51-2, die als Salz der Verbindung MAZ51 eine löslichere Variante mit ansonsten vergleichbaren Eigenschaften von MAZ51 darstellt.

In einer vorteilhaften Variante der vorliegenden Erfindung handelt es sich um Verbindungen, welche die Aktivität von Rezeptor-Tyrosin-Kinasen blockieren, die an der Angionese und/oder Lymphangiogenese beteiligt sind. In einer weiteren Variante hemmen die erfindungsgemäßen Verbindungen, einzeln oder deren Kombination, die Aktivität von Rezeptor-Tyrosin-Kinasen aus der Gruppe VEGFR-3 (Vascular Endothelial Cell Growth Factor Receptor) und/oder VEGFR-2. Eine vorteilhaften Variante der Erfindung umfaßt Verbindungen, welche die Aktivität des Zelloberflächenrezeptors VEGFR-3 blockieren.

Im Sinne der Erfindung hemmen die Verbindungen neben der natürlichen Aktivität von Proteinkinasen auch die Aktivität von pathologisch veränderten Proteinkinasen, d.h. von Proteinkinasen, deren Aktivität krankhaft außer Kontrolle geratenen ist. Beispielsweise können die Proteinkinasen dauerhaft (konstitutiv) stimuliert sein. Dies kann durch veränderte Eigenschaften im Interaktionsverhalten mit Liganden (Effektoren), oder anderen Proteinkomponenten oder eine veränderte Autostimulierung der RTKs ausgelöst worden sein.

Unter pathologisch veränderten Proteinkinase-Varianten sind im Sinne der vorliegenden Erfindung beispielsweise Proteinkinasen zu verstehen, die durch Veränderungen auf Nukleinsäure- und/oder Proteinebene eine unkontrollierte Zellvermehrung und/oder eine gestörte Apoptose von Zellen bewirken und/oder zu einer gestörten Angiogenese und/oder Lymphangiogenese und den damit verbundenen Krankheiten führen (siehe Tabelle 2) und/oder an der Krankheit Filariasis beteiligt sind. Unter Veränderungen auf Nukleinsäureebene sind erfindungsgemäß Mutationen zu verstehen, wozu z. B. Deletionen, Insertionen oder Austausche einer oder mehrerer Nukleotide zu verstehen sind. Unter Veränderungen auf Proteinebene sind Deletionen, Insertionen oder Austausche einer oder mehrerer Aminosäuren zu verstehen.

Das Wirkprinzip der erfindungsgemäßen Verbindungen beruht darauf, daß sie das natürliche Substrat der Proteinkinasen, beispielsweise ATP, vortäuschen. D.h. sie wirken sozusagen als ATP-Analoga. Dabei binden sie mit ihrem Oxindol-Anteil an die gleiche Domäne der Proteinkinasen, an die natürlicher Weise der Adeninring des ATPs bindet. Die erfindungsgemäßen Verbindungen und das natürliche Substrat konkurrieren somit um die Substratbindestelle, d.h. die erfindungsgemäßen Verbindungen verdrängen das ATP von der Substratbindestelle. Aufgrund dessen können die Proteinkinasen keinen Transfer einer Phosphatgruppe auf ein Zielmolekül katalysieren und so ihre bestimmungsgemäße Funktion als Kinase nicht mehr ausüben. Die erfindungsgemäßen Verbindungen sind somit aufgrund ihrer Struktur in der Lage nicht nur generell die Aktivität von Proteinkinasen zu hemmen, sondern auch menschliche Erkrankungen zu behandeln, die durch mutierte (konstitutiv) aktive RTKs hervorgerufen werden. Hierbei ist der Einsatz jeder einzelnen Verbindungen, aber auch deren Kombinationen denkbar.

Die erfindungsgemäßen Verbindungen weisen die Vorteile auf, daß es sich um kleine Moleküle handelt, die eine sehr gute Zell- bzw. Gewebepenetranz aufweisen. Ferner sind sie sehr gut zur Verabreichung am Patienten, bevorzugt oral, geeignet. Außerdem ist die Herstellung der Verbindungen auch im großtechnischen Maßstab für den pharmazeutischen Einsatz einfach und kostengünstig. Besonders vorteilhaft ist darüber hinaus, daß diese Verbindungen keine Immunantwort auslösen.

Gegenstand der vorliegenden Erfindung sind auch Mittel enthaltend wenigstens eine erfindungsgemäße Verbindung der zuvor genannten Art zur Behandlung von Erkrankungen, an deren Entstehung, Verlauf, Linderung und/oder Heilung natürlich vorkommende und/oder pathologisch veränderte Proteinkinasen beteiligt sind. Dies umfaßt erfindungsgemäß Mittel zur Hemmung der unkontrollierten Vermehrung und/oder Induzierung der Apoptose von Zellen und/oder zur Hemmung der Angiogenese und/oder Lymphangiogenese enthaltend wenigstens eine Verbindung der zuvor genannten Art. Ferner sind die erfindungsgemäßen Mittel enthaltend wenigstens eine erfindungsgemäße Verbindung zur Behandlung von Angiogenese- und/oder Lymphangiogenese-abhängigen Erkrankungen (wie z.B. in Tabelle 2 aufgeführt) und/oder Filriasis geeignet.
Die zuvor genannten Mittel zeichnen sich ferner dadurch aus, daß sie wenigstens eine Verbindung der erfindungsgemäßen Art in einer Konzentration von etwa 1-20, bevorzugt von etwa 2-15, besonders bevorzugt von etwa 3-10 und insbesondere von etwa 4-8 mg/kg Körpergewicht des Probanden enthalten. Denkbar sind auch Kombinationen der erfindungsgemäßen Verbindungen in frei kombinierbaren Konzentrationsanteilen der genannten Bereiche.

Erfindungsgemäß umfaßt sind auch Mittel enthaltend wenigstens eine Verbindung der zuvor genannten Art zur Regulierung der biologischen Funktion von Proteinen, die ihrerseits durch Proteinkinasen, die wiederum durch die erfindungsgemäßen Verbindungen gehemmt werden, hinsichtlich ihrer Aktivität kontrolliert werden (Signaltransduktion).

Darüber hinaus können die erfindungsgemäßen Mittel zusätzliche Stoffe enthalten, die zur besseren Verarbeitung oder Verabreichung am Patienten erforderlich der geeignet sind. Diese Stoffe ebenso wie die Herstellungsverfahren für die erfindungsgemäßen Mittel sind gängige Laborpraxis und werden deshalb hier nicht näher erläutert.

Gegenstand der vorliegenden Erfindung ist die Verwendung der Verbindungen der zuvor genannten Art zur Herstellung von Mitteln zur Hemmung der unkontrollierten Vermehrung und/oder Induzierung der Apoptose von Zellen und/oder zur Hemmung der Angiogenese und/oder Lymphangiogenese. Ebenso ist die Verwendung der erfindungsgemäßen Verbindungen zur Behandlung von Angiogenese- und/oder Lymphangiogenese-abhängigen Erkrankungen (Tabelle 2) und/oder Filariasis umfaßt.

Die nachfolgenden Beispiele dienen zur Erläuterung der vorliegenden Erfindung, wirken sich jedoch nicht limitierend auf die Erfindung aus:

### 1) Struktur und Synthese von MAE87, MAE106 und MAZ51

### Allgemeine Vorschrift:

10 mmol Oxoindol, 10 mmol Aldehyd und einige Tropfen Piperidin werden in 40 ml Ethanol gelöst. Die Reaktionsmischung wird 5 Stunden bei 90°C gerührt. Das gewünschte Produkt (E/Z-Gemisch) fällt während der Reaktion oder nach Abkühlen auf Raumtemperatur aus. Der Niederschlag wird abfiltriert, mit Ethanol gewaschen und im Vakuum getrocknet.

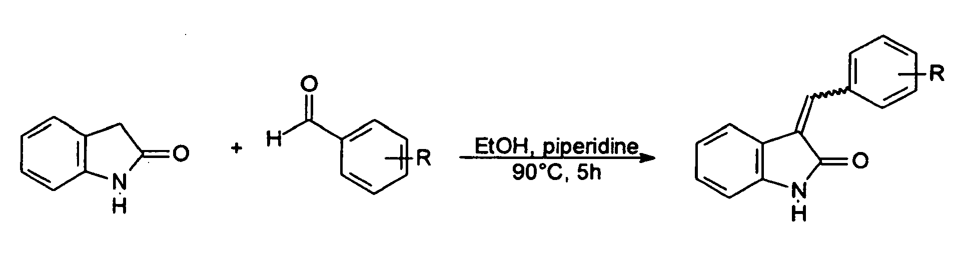

Die NMR Spektren wurden auf einem Bruker DRX500 Spektrometer aufgenommen. Die chemischen Verschiebungen sind in ppm (parts per million) angegeben. Als interner Standard dient das Restprotonensignal des Lösungsmittels. Hochauflösende Massenspektren wurden mit einem Finnigan MAT MS 70 Massenspektrometer aufgenommen. Die Schmelzpunkte sind nicht korrigiert.

### 1a) 3-(2,4-Dihydroxy-benzyliden)-1,3-dihydro-indol-2-on (MAE87)

Eine Mischung von 1,33 g Indol-2-on (10mmol), 1,38 g 2,4-Dihydroxybenzaldehyd (10mmol) und 3 Tropfen Piperidin werden in 40 ml Ethanol für 5 Stunden rückflussiert. Während der Reaktion fällt das gewünschte Produkt als gelber Feststoff aus. Nach Abkühlung auf Raumtemperatur wird das Produkt abfiltriert, mit Ethanol gewaschen und im Vakuum getrocknet. Ausbeute 1,28 g (51%).
Schmelzpunkt: 250°C Zersetzung
¹H-NMR (500 MHz, DMSO-d₆):
δ [ppm]: 6,38 (dd, J = 2 Hz, J = 8,5 Hz, 1H); 6,44 (d, J = 2 Hz, 1H); 6,85 (m, 2H); 7,17 (t, J = 7,5 Hz, 1H); 7,55 (d, J = 8,3 Hz, 1H); 7,63 (d, J = 7,6 Hz, 1H); 7,7 (s, 1H); 10,2 (br, 2H); 10,45 (s, 1H)
¹³C-NMR (125 MHz, DMSO-d₆):
δ [ppm]: 102,9; 107,4; 110,2; 113,1; 121,3; 122,2; 122,3; 123,5; 129,2; 131,2; 133,4; 142,5; 159,2; 161,7; 169,7
HR-MS: C₁₅H₁₁NO₃: ber.: m/z = 253,0739 gef.: m/z = 253,0744

### 1b) 3-(3-Fluoro-4-methoxy-benzyliden)-1,3-dihydro-indol-2-on (MAE106)

Eine Mischung von 1,33 g Indol-2-on (10mmol), 1,54 g 3-Fluor-4-methoxybenzaldehyd (10mmol) und 3 Tropfen Piperidin werden in 40 ml Ethanol für 5 Stunden rückflussiert. Nach Abkühlung auf Raumtemperatur fällt das gewünschte Produkt als gelber kristalliner Feststoff aus. Das Produkt wird abfiltriert, mit Ethanol gewaschen und im Vakuum getrocknet. Ausbeute 2,2 g (82%).
Schmelzpunkt: 220°C
¹H-NMR (500 MHz, DMSO-d₆, E/Z-Isomere):
δ [ppm]: 3,92 (s, 3H); 6,83 (d, J = 7,2 Hz, 0,7H); 6,87 (m, 0,7H); 6,98 (t, J = 7,6 Hz, 0,7 H); 7,18-7,32 (m, 2H); 7,54 (s, 0,3H); 7,6 (m, 1H); 7,65 (d, J = 7,6 Hz, 0,6H); 7,74 (s, 0,7 H); 8,0 (d, J = 8,3 Hz, 0,7 H); 8,80 (dd, J = 2 Hz, J = 13 Hz, 0,6 H); 10,65 (br, 1H)
¹³C-NMR (125 MHz, DMSO-d₆):
δ [ppm]: 109,8; 110,6; 113,6; 114,3; 117,4; 117;6; 118,9; 119,0; 119,9; 121,3; 121,5; 121,6; 122,6; 125,5; 125,7; 121,1; 127,2; 127,5; 127,6; 127,7; 127,8; 129,1; 130,5; 121,1; 135,1; 136,2; 140,9; 143,3; 148,7; 148,8; 149,5; 149,6; 150,0; 150,6; 151,9; 152,5; 167,8; 169,2
HR-MS: C₁₆H₁₂NO₂F: ber.: m/z = 298,9946 gef.: m/z = 298,9954

### 1c) 3-(4-Dimethylamino-naphthalen-1-ylmethylen)-1,3-dihydro-indol-2-on (MAZ51)

Eine Mischung von 1,33 g Indol-2-on (10mmol), 1,99 g 4-Dimethylamino-1-naphtaldehyd (10mmol) und 3 Tropfen Piperidin werden in 40 ml Ethanol für 5 Stunden rückflussiert. Nach Abkühlung auf Raumtemperatur fällt das gewünschte Produkt als orangener Feststoff aus. Das Produkt wird abfiltriert, mit Ethanol gewaschen und im Vakuum getrocknet. Ausbeute 2,67 g (85%).
¹H-NMR (500 MHz, DMSO-d₆):
δ [ppm] : 2,90 (s, 6 H; (CH₃)₂) 6,74 (dt, J = 1 Hz, J = 7,5 Hz, 1 H); 6,87 (d, J = 7,5 Hz, 1 H); 7,15 (m, 2 H); 7,24 (d, J = 8 Hz, 1 H); 7,59 (m, 2 H); 7,83 (d, J = 8 Hz, 1 H); 7,93 (m, 1 H); 8,06 (s, 1 H); 8,23 (m, 1 H); 10,64 (br, 1 H, -NH).
¹³C-NMR (125 MHz, DMSO-d₆):
δ [ppm]: 45,1; 110,4; 113,4; 121,4; 121,7; 122,7; 125,2; 125,3; 125,4; 126; 127,4; 128,1; 128,2; 128,3; 130,2; 132,8; 134,1; 143,2; 153,1; 169
HR-MS: C₂₁H₁₈N₂O: ber.: m/z = 314,1419 gef.: m/z = 314,1419
Schmelzpunkt: 250°C Zersetzung

### 2) MAE87, MAE106 und MAZ51 inhibieren eine breite Anzahl von Rezeptor-Tyrosin-Kinasen im in vitro Tyrosin-Kinase-Assay.

| Inhibitor | | Kinase | | | | | | |
|---|---|---|---|---|---|---|---|---|
| Name | Konz. | VEGFR-3 | VEGFR-2 | Tie2 | EGFR | ErbB2 | IGF1R | FGFR1 |
| MAE87 | 1 µg/m | -9 | -46 | +1 | -70 | -45 | -63 | -50 |
| | 10µg/ml | -74 | -77 | -65 | -86 | -84 | -94 | -94 |
| MAE106 | 1µg/ml | +1 | +21 | -5 | -5 | +4 | -10 | +5 |
| | 10µg/ml | -57 | -58 | -28 | -64 | -41 | -71 | -1 |
| MAZ51 | 1µg/ml | -4 | -8 | -7 | -25 | -11 | -10 | +1 |
| | 10µg/ml | -35 | -74 | -27 | -58 | -39 | -68 | -5 |

Die Hemmung verschiedener Rezeptor-Tyrosin-Kinasen in vitro durch MAE87, MAE106 und MAZ51 wurde untersucht. Die Inhibitoren wurden in 2 verschiedenen Konzentrationen (1µg/ml und 10µg/ml) untersucht. Die Inhibition ist in % angegeben. "+" = Stimulation der Kinaseaktivität relativ zur Kontrolle,
"-" = Inhibition der Kinaseaktivität relativ zur Kontrolle.

Die Aktivität der RTK-Inhibitoren wurden mit Hilfe eines ELISAs bestimmt. In diesem Assay werden die entsprechenden Kinasen als rekombinante GST-Fusionsproteine (Glutathione S-Transferase) eingesetzt. Als Akzeptor dient synthetisches polyGlu, Tyr 4:1. Die Fähigkeit verschiedener Konzentrationen der Testsubstanzen die RTK-vermittelte Phosphorylierung des o. g. Akzeptors zu inhibieren wurde bewertet. Alle Tests wurden in Doppelbestimmung durchgeführt.
ELISA-Platten wurden über Nacht mit 0,2 mg/ml polyGlu, Tyr 4:1 in 100 mM Bicarbonat-Puffer (pH 9,6) beschichtet. Diese Lösung wurde entfernt und die Mikrotiterplatten mit TBS-Puffer (10 mM Tris-HCl, pH8.1, 100 mM NaCl) zweimal gewaschen, und anschließend mit 5% BSA/TBS 30 min. geblockt. 50µl Testsubstanz (2 oder 20 µg/ml in 10% DMSO), 25 µl GST-Kinase in 4x Kinase-Puffer (200 mM HEPES, 100 mM NaCl, 80 µM Na₃VO₄ und 0.04% BSA) wurden vorgelegt. Die Reaktion wurde durch Zugabe von 25 µl 160 µM ATP (in 40 mM MnCl₂) als Substrat der Kinasen gestartet. Die Endkonzentrationen der Testverbindungen ist damit 1 oder 10 µg/ml in 5% DMSO. Die GST-Fusionsproteine wurden in folgenden Konzentrationen eingesetzt. VEGFR-2 50 NG/well, VEGFR-3 300 ng/well, Tie2 300 ng/well, EGFR 50 ng/well, ErbB2 200 ng/well, IGF-1 R 50 ng/well, FGFR1 200 ng/well. Die Reaktionsmischung wurde 90 min. bei 30°C inkubiert. Die Kinasereaktion wurde durch Zugabe von 50 µl/well 30 mM EDTA gestoppt. Die Mikrotiterplatten wurden zweimal mit 0,05% Tween20/TBS gewaschen. Anti-Phosphotyrosin Antikörper (1:500) wurde in 0,05% Tween20/TBS ( mit 0.5% BSA, 0.025% Magermilchpulver und 100 µM Na₃VO₄) zugegeben und 1 h bei 37°C inkubiert. Die Mikrotiterplatten wurden dreimal mit 0,05% Tween20/TBS gewaschen. Anschließend wurde der HRP-konjugierte Nachweisantikörper (1:1000) in 0,05% Tween20/TBS ( mit 0.5% BSA, 0.025% Magermilchpulver und 100 µM Na₃VO₄) zugegeben und für 1 h bei 37°C inkubiert. Die Mikrotiterplatten wurden dreimal mit 0,05% Tween20/TBS gewaschen. ABTS (2,2'-Azino-di-(3-ethylbenzthiazolin-6-sulphonat)) Substrat (Roche Diagnostics GmbH, Mannheim) wurde zugegeben. Die OD wurde mit einem ELISA-reader bei 405 nm bestimmt.

### 3) MAE87, MAE106 und MAZ51 inhibieren die Liganden-induzierte Autophosphorylierung der RTKs VEGFR-2 und VEGFR-3

VEGFR-2 bzw. VEGFR-3 exprimierende PAE Zellen (Pig Aorta Endothel Cells) wurden mit 0,5 µM, 5 µM oder 50 µM der entsprechenden Testverbindung inkubiert. Die Zellen wurden dann mit VEGF (VEGFR-2) oder VEGF-C (VEGFR-3) bzw. ohne Wachstumsfaktor als Negativkontrolle stimuliert. Nach 15 min wurden die Zellen geerntet und mit VEGFR-2 oder VEGFR-3 immunopräzipitiert. Die Immunopräzipitate wurden nach Elektrophorese geblottet und mit anti-Phosphotyrosin Antikörper untersucht. Die Blots wurden abgezogen und zur Ladungskontrolle mit VEGFR-2 oder VEGFR-3 Antikörper untersucht. Bei 5µM inhibiert MAZ51 selektiv VEGFR-3.

PAE/VEGFR-2 oder PAE/VEGFR-3 wurden in 15 cm Gewebekulturschalen ausgesäht und bis 50% Konfluenz kultiviert. Die Zellen wurden dann 16-24 h (PAE/VEGFR-3) oder 72 h (PAE/VEGFR-3) in serumfreiem Medium (mit 0,2% BSA) gehungert. Nach 30-60 min Vorinkubation mit 5ml 1mM serumfreiem Medium (1 mM Na₃VO₄) und verschiedenen Inhibitorkonzentrationen wurden die Zellen 5 min. (VEGFR-3) oder 8 min. (VEGFR-2) bei 37°C stimuliert. Danach wurden die Zellen schnell zweimal mit eiskaltem PBS gewaschen ( mit 1mM Na₃VO₄.) und mit eiskaltem modifiziertem RIPA Puffer (30 mM Tris-HCl, pH7.4, 150 mM NaCl, 1 mM EDTA, 0.5% (*v*/*v*) Triton X100, 0.5% *(wlv)* Natriumdesoxycholat, 10 mM NaF) frisch zubereitet mit 1 mM PMSF, 0.1 U/ml Aprotinin, 10 ng/ml Leupeptin und 5 mM Na₃VO₄ lysiert. Die Zellen werden von den Platten mit Hilfe eines Rubber-Policeman geerntet und in Zentrifugenröhrchen auf Eis gesammelt. Die Lysate wurden zur Solubilisierung durch eine Spritze mit einer 25G Kanüle gedrückt und zur Abtrennung unlöslicher Bestandteile bei 4°C/13000rpm 15 min zentrifugiert.

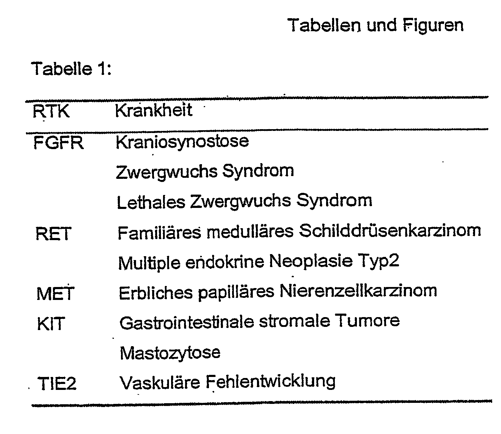

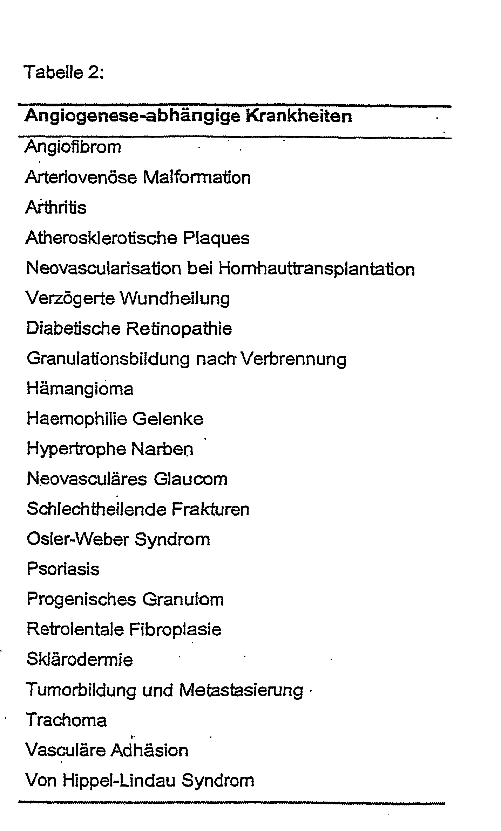

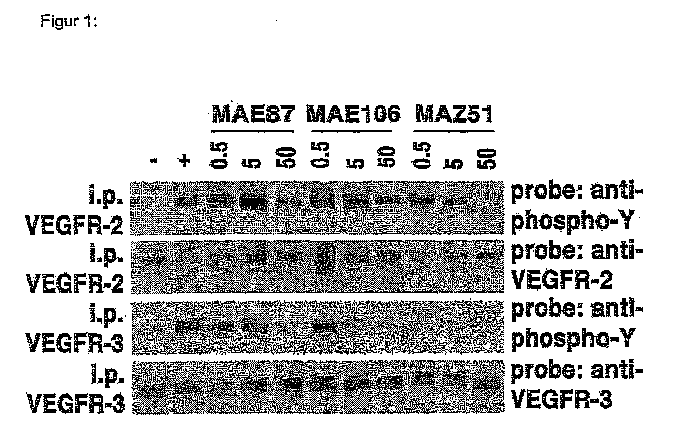

Die klaren Lysate wurden über Nacht mit 4µg anti-VEGFR-2 (C-1158, Santa Cruz) oder anti-VEGFR-2 Antikörper (M20, Santa Cruz) bei 4°C inkubiert.
Zum Ausfällen des Rezeptor-Antikörper-Komplexes wurden 30µl/Röhrchen Protein A-Sepharose (Amersham) zugegeben und für weitere 2 Stunden bei 4°C inkubiert.
Die Sepharose wurden dann 1 min. bei 4°C zentrifugiert und dreimal mit kaltem Waschpuffer Puffer (30 mM Tris-HCl, pH7.4, 150 mM NaCl, 1 mM EDTA, 0.5% *(vlv)* Triton X100, 0.5% (*w*/*v*) Natriumdesoxycholat, 10 mM NaF) frisch zubereitet mit 1 mM PMSF, 0.1 U/ml Aprotinin, 10 ng/ml Leupeptin und 5 mM Na₃VO₄ gewaschen. Der Rest des Waschpuffers wurde durch Absaugen durch eine, mit einer 27G Kanüle versehenen, Spritze entfernt. Die Sepharosekügelchen wurden in 50 µl SDS-Ladepuffer resuspendiert , aufgekocht und auf ein 6%iges Agarosegel geladen. Nach der elektrophoretische Auftrennung wurden die Proteine mittels Western Blot auf einer entsprechenden Membran fixiert. Die Blots wurden zuerst mit Hilfe eines anti-Phosphotyrosin Antikörpers (RC20:HRPO, Becton Dickinson) untersucht, dann wurde der erste Antikörper wieder entfernt um den Blot mit spezifischen anti-Rezeptor Antikörpern auf die Proteinladung zu untersuchen. Zum Ablösen des ersten Antikörpers wurde die Membranen mit Ablösepuffer (62.5 mM Tris, pH 6.8, 2% SDS, 0.75% 2-Mercaptoethanol) bei 55°C 20 min geschüttelt. Die Membranen wurden dann zweimal mit TBST je 2 min gewaschen und wie üblich geblockt und inkubiert.
Das Ergebnis ist in Figur 1 dargestellt.

### 4) MAE87, MAE106 und MAZ51 inhibieren die Endothelzell-Proliferation

Menschliche Nabelschnurvenen-Endothelzellen (HUVEC) wurden 24 h unter Serumentzug kultiviert. Dann wurden die Zellen 2 h mit den entsprechenden Verbindungen vorinkubiert und anschließend für weiter 24 h mit VEGF (MAE87) oder bFGF (MAE106, MAZ51) in Gegenwart der Inhibitoren kultiviert. Die Zellen wurden dann mit ³H-Thymidin inkubiert. Die in die zelluläre DNA inkorporierte Menge Radioaktivität wurde gemessen. Alle Experimente wurden in Dreifachbestimmung durchgeführt.

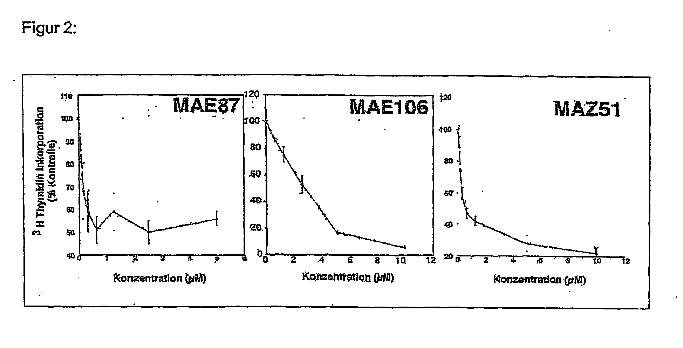

HUVE-Zellen wurden in 100 µl/well (10⁵ Zellen/ml) in 96-well Zellkulturplatten ausgesäht und über Nacht ruhen lassen. Die Zellen wurden dann für 24 h mit 50 µl/well serumfreiem Medium gehungert. Nachfolgend wurden 50 µl verschiedener Konzentrationen der Testsubstanzen in serumfreiem Medium (enthält 2% DMSO) zugegeben und 2 h bei 37°C inkubiert. Das die Testverbindungen enthaltene Medium wurde entfernt und durch 50 µl frisches Medium mit bFGF (12,5 ng/ml) oder VEGF (100 ng/ml) ersetzt. Frische Verdünnungen der zweifach konzentrierten Testsubstanzen in serumfreiem Medium mit 2% DMSO wurden in 50µl/well zu den Zellen gegeben. Die Endkonzentration DMSO war immer 1%. Nach 24 h wurde ³H-Thymidin (1 µCi/well) zugegeben und die Zellen wurden für weitere 4-6h inkubiert. Zur Analyse der inkorporierten Radioaktivität wurden die Zellen 30 min trypsiniert und mit Hilfe eines Harvester 96 cell (Tomtec) geerntet und auf einem Glasfasermembran-Filter fixiert. Die immobilisierte Radioaktivität wurde mit Hilfe eines MicroBeta TriLux Flüssigszintillations-Lumineszenzzählgeräts quantifiziert. Das Ergebnis ist in Figur 2 dargestellt.

### 5) MAE87, MAE106 und MAZ51 inhibieren die Angiogenese im CAM-Assay

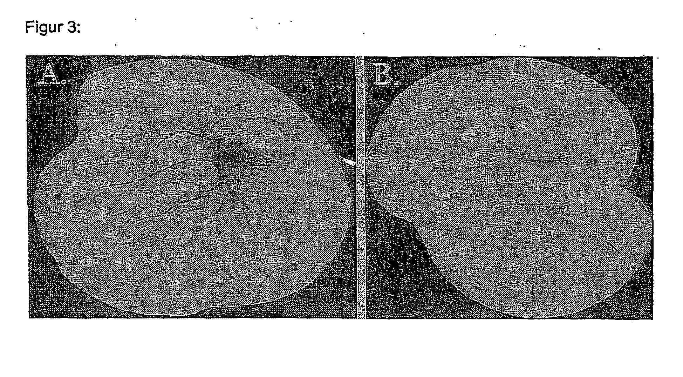

Der Chorioallantoismembran-(CAM)-Assay wurde wie beschrieben (Wernert et al.) mit Hühnerembryonen 5 Tage nach der Befruchtung durchgeführt. Methylcelluloseplättchen (Durchmesser 2mm) mit 100µg Inhibitor wurden auf die CAMs appliziert. Die Auswertung erfolgte am Tag 7. Repräsentative Ergebnisse sind für MAE106 in Figur 3 gezeigt. A. Negativkontrolle B. MAE106 behandeltes Ei. Das stark verzweigte Netzwerk von Blutgefäßen, das die Negativkontrolle zeigt, ist im MAE 106 behandelten Ei stark unterentwickelt.

### 6) MAE87, MAE106 und MAZ51 inhibieren die Proliferation von Tumorzellen

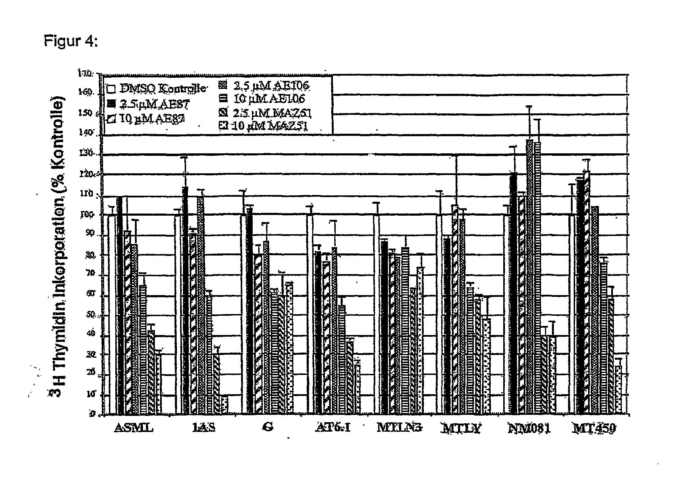

Die Zellen der Ratten Tumorzelllinie (Nestl et al., 2001, Cancer Research 61: 1569-1577) wurden 24 h in der Anwesenheit von 1% DMSO (Lösungsmittel Kontrolle), 2,5 µM oder 10 µM des Indolinons kultiviert. Tritiiertes Thymidin wurde dem Medium während der letzten 4-6 h der Inkubation zugefügt. Die Zellen wurden geerntet und die Menge an, in die DNA inkorporierter, Radioaktivität wurde quantifiziert. Die Daten sind in Prozent bezüglich der Proliferation der nur mit DMSO behandelten Zellen angegeben (% bezüglich Kontrolle; siehe Figur 4)). MAZ51 hatte den stärksten Inhibitorischen Effekt.

### 7) MAE87, MAE106 und MAZ51 induzieren Apoptose in Endothelzellen und Tumorzellen

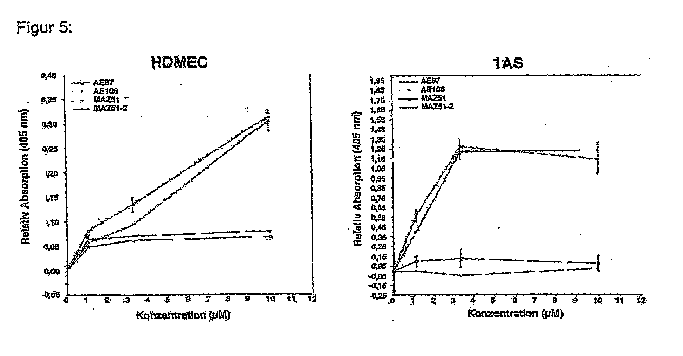

Menschliche Endothelzellen (HDMEC) und Ratten Pankreas-Tumorzellen (1AS) wurden resuspendiert (10⁵ Zellen/ml). 50 µl Zellsuspension wurden in 96-well Zellkulturplatten ausgesäht und 24h bei 37°C inkubiert. Die Zellen wurden dann mit den angegebenen Konzentrationen der verschiedenen Inhibitoren für weitere 24h inkubiert. Der pro-apoptotische Effekt der Verbindungen wurde mit dem Cell Death Detection ELISA^{plus} kit (Roche Diagnostics GmbH, Mannheim) nach Angaben des Herstellers bestimmt. Das kit enthält einen photometrischen Enzym-Immunoassay für die qualitative und quantitative in vitro Bestimmung von, durch aktiven Zelltod (Apoptose) freigestzten, cytoplasmatischen Histon-assoziierten DNA-Fragmenten (Mono- und Oligonucleosome). Über die Bestimmung der Optischen Dichte bei 405 nm läßt sich der Anteil an induzierter Apoptose quantifizieren. Die Meßwerte bezogen auf die unbeandelten Zellen sind gegen die Inhibitorkonzentration aufgetragen. Die Ergebnisse in Figur 5 zeigen, daß MAZ51 ein potenter Aktivator der Apoptose sowohl in Endothelzellen als auch Tumorzellen ist.

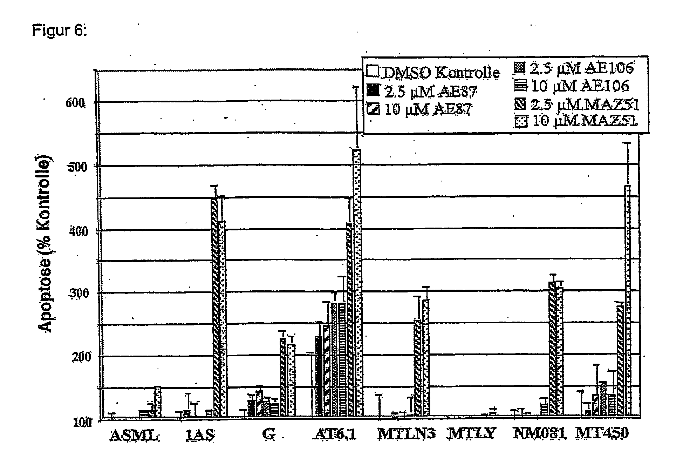

Die Zellen von der Ratten Tumorzelllinie (Nestl et al., 2001, Cancer Research 61: 1569-1577) wurden 24 h in der Anwesenheit von 1% DMSO (Lösungsmittel Kontrolle), 2,5 µM oder 10 µM des Indolinons kultiviert. Apoptose wurde mit Hilfe von anti-DNA-Peroxidase Antikörper quantifiziert. Die Menge an Farbstoff, der in einer durch die Peroxidase katalysierten chromogenen Reaktion erzeugt wird, wurde photometrisch bei 405nm gemessen und mit der Menge an Apoptose-assoziierten cytoplasmatischen Mono- und Oligonucleosomen korreliert. Die Daten sind in % Apoptose bezüglich der Zellen der Lösungsmittelkontrolle (% bezüglich Kontrolle) angegeben (Figur. 6). MAZ51 ist der potenteste Apoptose-Induktor.

### 8) MAZ51 inhibiert das Wachstum von 1AS und MT450 Ratten-Karzinomen in vivo.

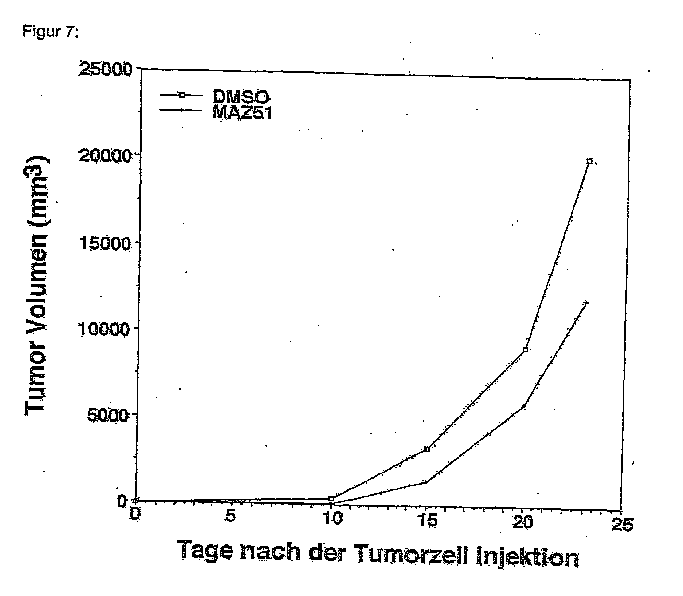

1AS Zellen (5x10⁵) wurden subkutan in 2 Gruppen von BDX-Ratten (8 Ratten pro Gruppe) injiziert. Einer Gruppe wurde nachfolgend täglich 100µl/Tier DMSO bis zum Abschluß des Experiments injiziert. Der anderen Gruppe wurden täglich 100µl/Tier MAZ51 (10mg/ml in DMSO), entsprechend 4-5 mg/kg, injiziert. Das Tumorvolumen wurde regelmäßig nach der Injektion der Tumorzellen gemessen. Wie anhand von Figur 7 zu sehen ist, wird durch die Behandlung mit MAZ51 das Wachstum von AS1-Tumoren in vivo wesentlich gehemmt.

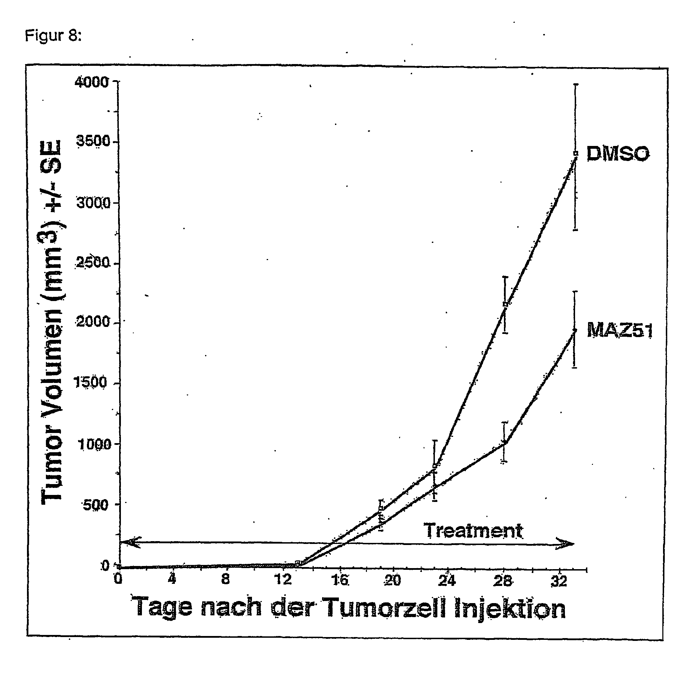

MT450 Ratten Mammakarzinomzellen wurden subkutan in Wistar Furth Ratten injiziert. Die Wirkstoffbehandlung mit 8mg/kg/Tag MAZ51 in 100% DMSO oder Lösungsmittelkontrolle (100% DMSO) wurde am Tag nach der Injektion der Tumorzellen begonnen. MAZ51 oder nur Lösungsmittel wurde täglich intraperitoneal injiziert. Jede Testgruppe umfaßte 8 Tiere. Die Tumore wurden alle 4-5 Tage vermessen. Die Mittleren Tumorvolumen sind Figur 8 angegeben.

### Legende zu den Tabellen und Figuren:

- Tabelle 1:: Übersicht über Aktivierungsmutationen verschiedener Rezeptor-Tyrosin-Kinsasen und die dadurch ausgelösten Krankheiten.
- Tabelle 2:: Übersicht über Angiogensese-abhängige Krankheiten
- Figur 1:: Western-Blot von Immunpräzipitationen (i.p.) von VEGFR-2 und VEGFR-3 aus PAE-Zellen, die mit MAE87, MAE106 und MAZ51 behandelt wurden. (-) stellt die Kontrolle dar (nicht stimulierte Zellen); (+) sind Wachstumsfaktor-stimulierte Zellen, die ohne bzw. mit 0,5 µM, 5 µM oder 50 µM des entsprechend angegebenen Inhibitors (MAE87, MAE106 oder MAZ51) behandelt wurden. Die zur Immunpräzipitation eingesetzten Antikörper-Proben (probe) ist entweder Anti- Phosphotyrosin-Antikörper (anti-phospho-Y), Anti-VEGFR-2- Antikörper (anti-VEGFR-2) oder Anti-VEGFR-3-Antikörper (anti-VEGFR-3).
- Figur 2:: Messung des (³H)-Thymidineinbaus in humane endotheliale Zellen (HUVEC) in Abhängigkeit von der Konzentration an eingesetzten Inhibitoren (MAE87, MAE106 und MAZ51). Die Hemmung der Zellproliferation durch MAE87, MAE106 und MAZ51 ist dabei Dosis-abhängig.
- Figur 3:: Hemmung der Angiogenese in einer Chorioallantois Membran (CAM). A: Scheinbehandelte Zellen (Kontrolle). B: Mae106-behandelte Zellen der CAM.
- Figur 4:: Messung des (³H)-Thymidineinbaus (in %) in Ratten- Tumorzellinien (ASML, 1AS, G, AT6.1, MTLN3, MTLY, NM081 und MT450) in Abhängigkeit von der Konzentration an eingesetzten Inhibitoren. Die Hemmung der Zellproliferation durch MAE87, MAE106 und MAZ51 ist Dosis-abhängig.
- Figur 5:: Nukleosom-ELISA-Test (Cell Death Detection ELISA). Dargestellt ist das Ausmaß abgestorbener Zellen der jeweiligen Zelline (als relative Absorption bei 405 nm) im Verhältnis zum Einsatz der Inhibotoren MAE87, MAE106 und MAZ51 und MAZ51-2. MAZ51 induziert den Zelltod in humanen endothelialen Zellen (HDMEC) und der Ratten Pankreaszellinie 1AS am effektivsten.
- Figur 6:: Nukleosom-ELISA-Test (Cell Death Detection ELISA). Dargestellt ist das Ausmaß abgestorbener Zellen der jeweiligen Ratten Tumorzelline (Apoptosis in %) im Verhältnis zum Einsatz der Inhibotoren MAE87, MAE106 und MAZ51. MAE87, MAE106 und MAZ51 induzieren den Zelltod in einer Reihe von Ratten-Karzinomzellinien.
- Figur 7:: Darstellung des Tumorvolumens in Abhängigkeit von der Anzahl an Tagen nach der Tumorzellinjektion in Abhängigkeit von der Behandlung der Tumorzellen 1AS mit MAZ51. Die untersuchten Ratten zeigen, daß nach Behandlung der 1AS- Tumorzellen mit MAZ51 das Tumorwachstum in vivo gehemmt wird.
- Figur 8:: Darstellung des Tumorvolumens in Abhängigkeit von der Anzahl an Tagen nach der Tumorzellinjektion in Abhängigkeit von der Behandlung der Tumorzellen MT450 mit MAZ51 und einer DMSO-Lösung (Negativkontrolle). Die untersuchten Ratten zeigen, daß nach Behandlung mit MAZ51 das Tumorwachstum der MT450-Tumorzellen in vivo gehemmt wird. +/-SE bedeutet die Standardabweichung des Tumorvolumens vom Mittelwert von 8 untersuchten Ratten pro Ansatz.

## Patentansprüche

1. Mittel zur Blockierung der Aktivität von Rezeptor-Tyrosin-Kinasen aus der Gruppe VEGFR-3 (Vascular Endothelial Cell Growth Factor Receptor) und/oder VEGFR-2 enthaltend wenigstens eine Verbindung gemäß der Strukturformel I) 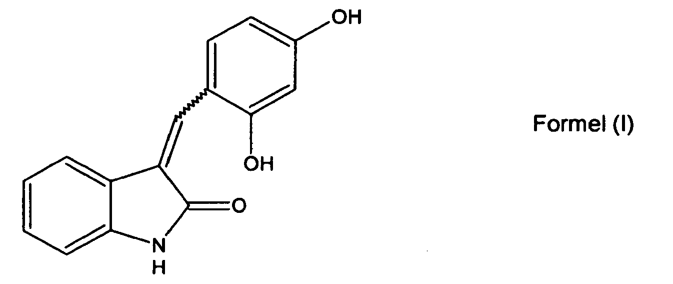 oder der Strukturformel (II) 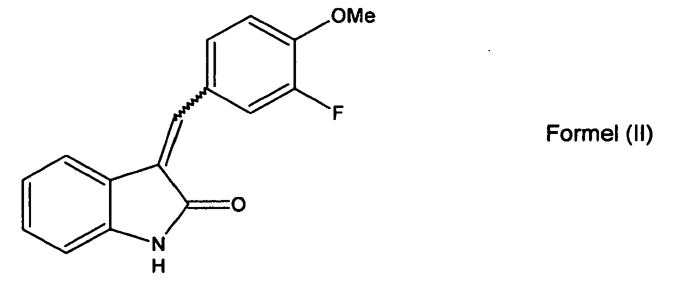 oder der Strukturformel (III) 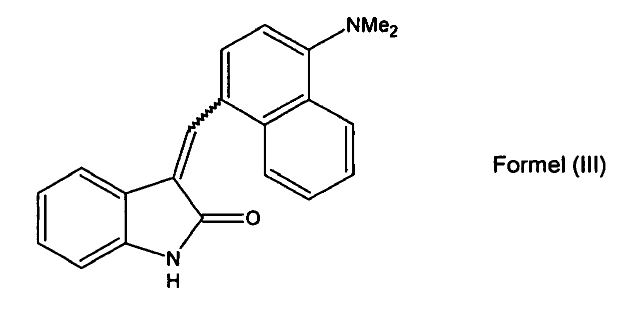 oder deren physiologisch verträgliche Salze.

2. Mittel gemäß Anspruch 1 zur Hemmung der Angiogenese und/oder Lymphangiogenese.

3. Mittel gemäß einem der Ansprüche 1 oder 2 zur Behandlung von Angiogeneseabhängigen und/oder Lymphangiogenese-abhängigen Krankheiten und/oder Filariasis.

4. Mittel gemäß einem der Ansprüche 1 bis 3 zur Hemmung der unkontrollierten Vermehrung und/oder Induzierung der Apoptose von Zellen.

5. Mittel gemäß einem der Ansprüche 1 bis 4 zur Blockierung der Aktivität des Zelloberflächenrezeptors VEGFR-3.

6. Mittel gemäß einem der Ansprüche 1 bis 5, **dadurch gekennzeichnet, daß** es wenigstens eine Verbindung gemäß der Strukturformel I), II) oder III) oder deren physiologisch verträgliche Salze in einer Konzentration von etwa 1-20, bevorzugt von etwa 2-15, besonders bevorzugt von etwa 3-10 und insbesondere von etwa 4-8 mg/kg Körpergewicht des Probanden enthält.

7. Verwendung der Verbindungen gemäß der Strukturformel I) 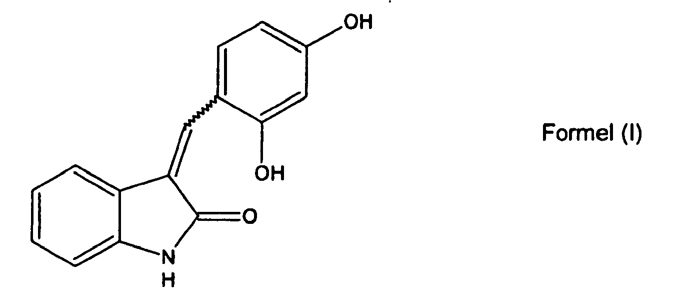 oder der Strukturformel (II) 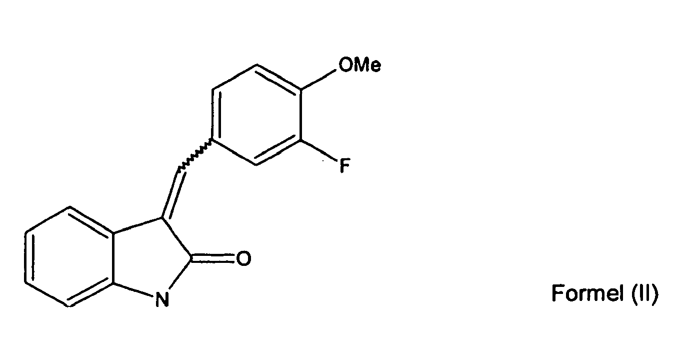 oder der Strukturformel (III) 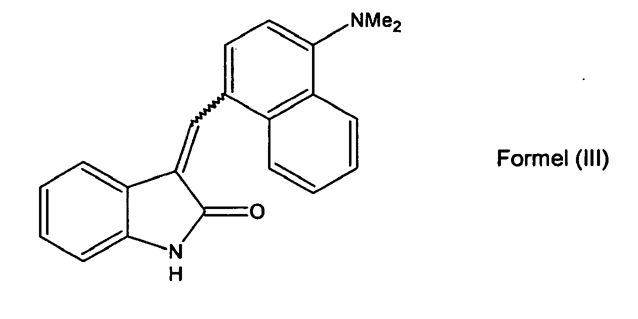 der deren physiologisch verträgliche Salze zur Herstellung von Mitteln zur Hemmung der unkontrollierten Vermehrung und/oder Induzierung der Apoptose von Zellen und/oder zur Hemmung der Angiogenese und/oder Lymphangiogenese.

## Claims

1. Composition for blocking the activity of receptor tyrosine kinases from the group consisting of VEGFR-3 (Vascular Endothelial Cell Growth Factor Receptor) and/or VEGFR-2, comprising at least one compound according to the structural formula I) 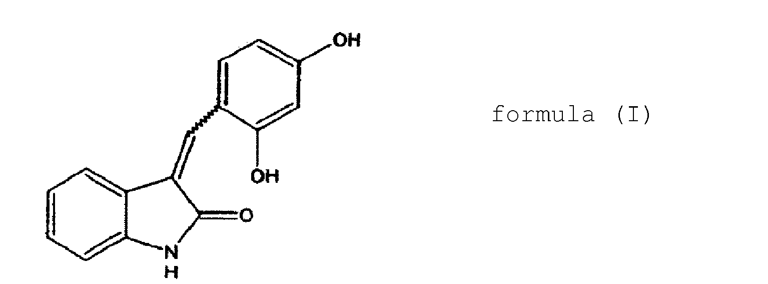 or of the structural formula (II) 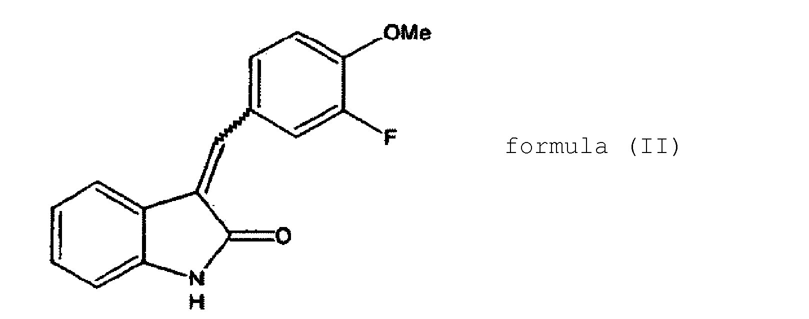 or of the structural formula (III) 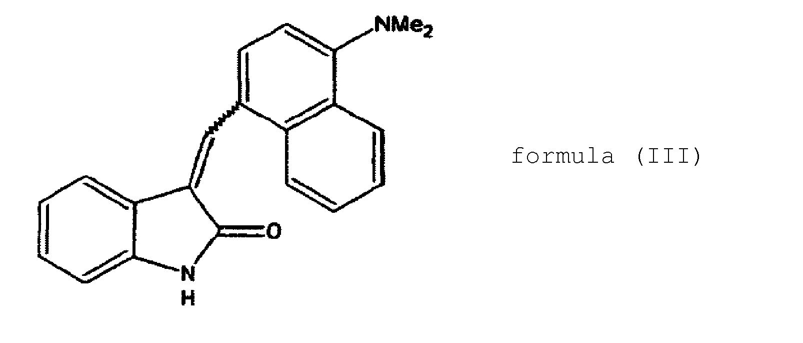 or the physiologically acceptable salts thereof.

2. Composition according to Claim 1 for inhibiting angiogenesis and/or lymphangiogenesis.

3. Composition according to either of Claim 1 or 2 for the treatment of angiogenesis-dependent and/or lymphangiogenesis-dependent diseases and/or filariasis.

4. Composition according to any of Claims 1 to 3 for inhibiting uncontrolled proliferation and/or inducing apoptosis of cells.

5. Composition according to any of Claims 1 to 4 for blocking the activity of the cell surface receptor VEGFR-3.

6. Composition according to any of Claims 1 to 5, **characterized in that** it comprises at least one compound according to the structural formula I), II) or III) or the physiologically acceptable salts thereof in a concentration of about 1-20, preferably of about 2-15, particularly preferably of about 3-10 and in particular of about 4-8, mg/kg of the subject's body weight.

7. Use of the compounds according to the structural formula I) 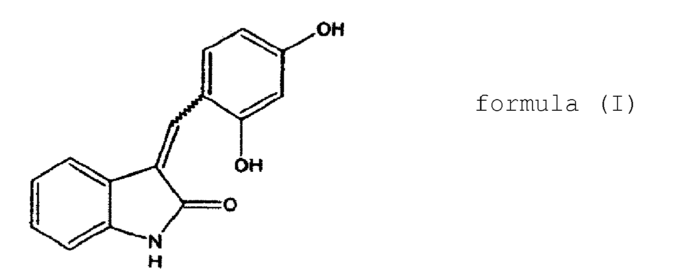 or of the structural formula (II) 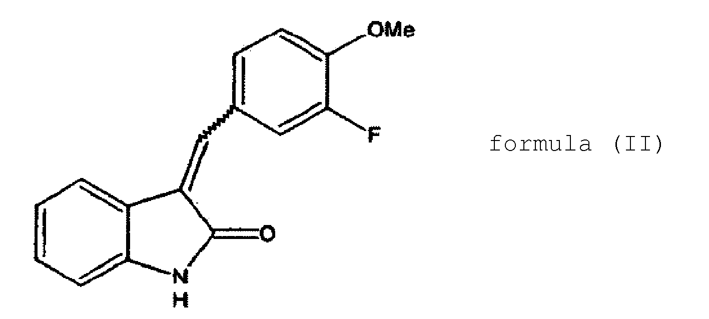 or of the structural formula (III) 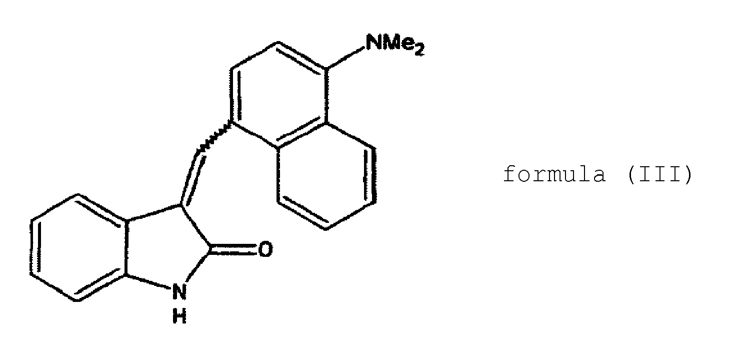 or the physiologically acceptable salts thereof for producing compositions for inhibiting uncontrolled proliferation and/or inducing apoptosis of cells and/or for inhibiting angiogenesis and/or lymphangiogenesis.

## Revendications

1. Agent destiné au blocage de l'activité de récepteurs à tyrosine kinase choisis dans le groupe constitué par VEGFR-3 (Vascular Endothelial Cell Growth Factor Receptor) et/ou VEGFR-2, contenant au moins un composé selon la formule développée (I) 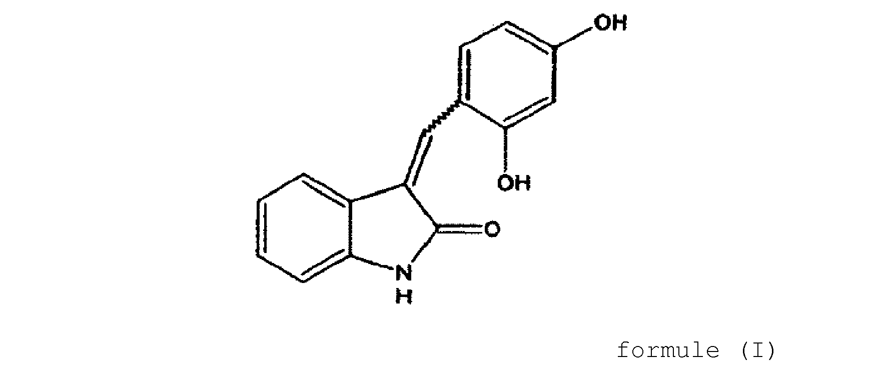 ou la formule développée (II) 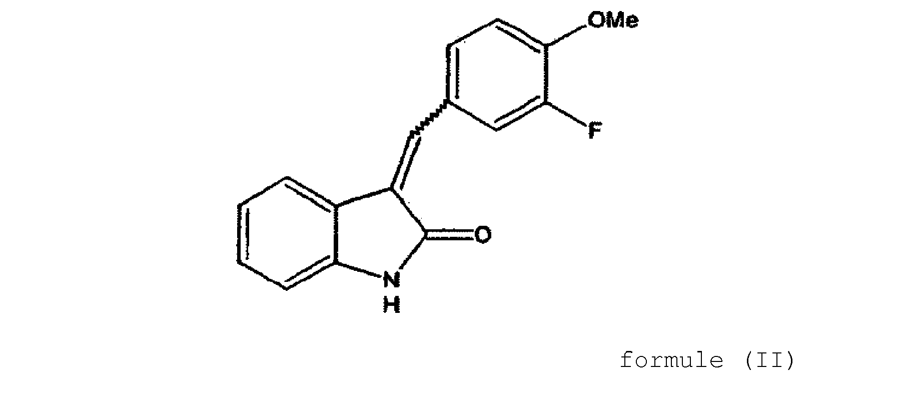 ou la formule développée (III) 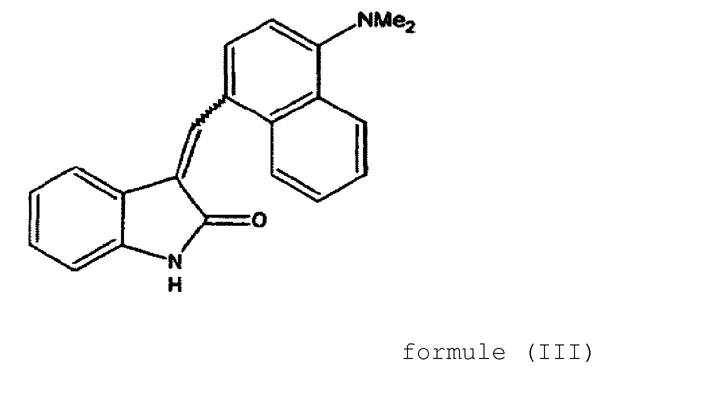 ou ses sels physiologiquement acceptables.

2. Agent selon la revendication 1, destiné à l'inhibition de l'angiogenèse et/ou de la lymphangiogenèse.

3. Agent selon la revendication 1 ou 2, destiné au traitement de la filariose et/ou de maladies dépendant de l'angiogenèse et/ou dépendant de la lymphangiogenèse.

4. Agent selon l'une quelconque des revendications 1 à 3, destiné au traitement de la multiplication incontrôlée et/ou de l'induction incontrôlée de l'apoptose de cellules.

5. Agent selon l'une quelconque des revendications 1 à 4, destiné au blocage de l'activité du récepteur de surface cellulaire VEGFR-3.

6. Agent selon l'une quelconque des revendications 1 à 5, **caractérisé en ce qu'**il contient au moins un composé selon la formule développée I), II) ou III) ou ses sels physiologiquement acceptables, à une concentration d'environ 1-20, de préférence d'environ 2-15, de façon particulièrement préférée d'environ 3-10 et en particulier d'environ 4-8 mg/kg de poids corporel du sujet.

7. Utilisation des composés selon la formule développée (I) 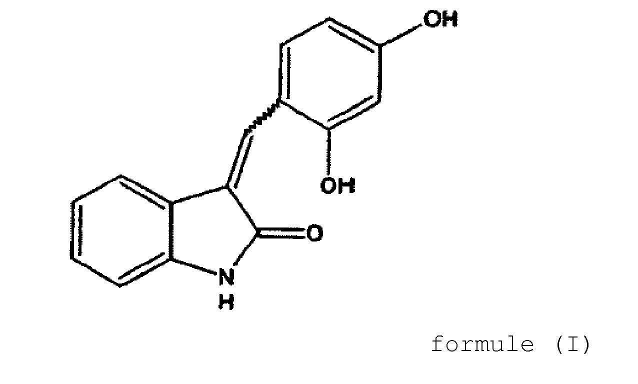 ou la formule développée (II) 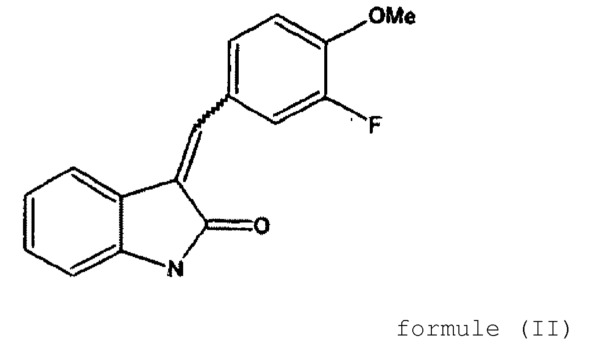 ou la formule développée (III) 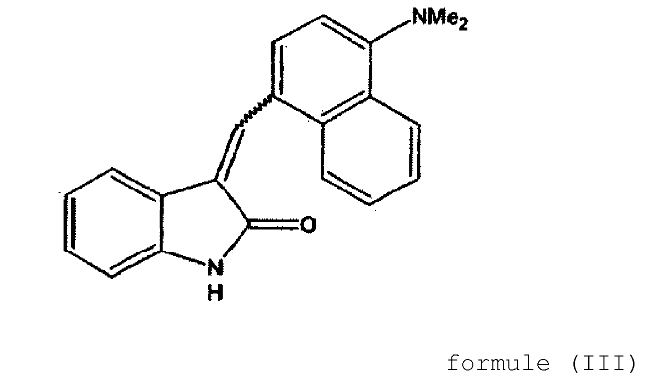 ou de leurs sels physiologiquement acceptables, pour la préparation d'agents destinés à l'inhibition de la multiplication incontrôlée et/ou de l'induction incontrôlée de l'apoptose de cellules et/ou à l'inhibition de l'angiogenèse et/ou de la lymphangiogenèse.
